# EUROPÄISCHE PATENTSCHRIFT

(11) **EP 3 866 901 B1**
(45) Veröffentlichungstag und Bekanntmachung des Hinweises auf die Patenterteilung: **26.11.2025**
(21) Anmeldenummer: 19755865.3
(22) Anmeldetag: 13.08.2019
(51) Int. Cl.: A61M 25/02

(54) **FIXIERVORRICHTUNG ZUR FIXIERUNG EINER KATHETERANORDNUNG AN EINEM PATIENTEN**
FIXING DEVICE FOR FIXING A CATHETER ASSEMBLY TO A PATIENT
DISPOSITIF DE FIXATION DESTINÉ À FIXER UN ENSEMBLE CATHÉTER SUR UN PATIENT

(30) Priorität: 17.10.2018 DE 102018217784
(43) Veröffentlichungstag der Anmeldung: 25.08.2021
(73) Patentinhaber: B. Braun Melsungen AG, 34212 Melsungen (DE)
(72) Erfinder: WIEGEL, Heinz, 36211 Alheim (DE)
(74) Vertreter: Patentanwälte Ruff, Wilhelm, Beier, Dauster & Partner mbB
(86) Internationale Anmeldenummer: PCT/EP2019/071686
(87) Internationale Veröffentlichungsnummer: WO 2020/078601

(56) Entgegenhaltungen:
- US-A1- 2005 038 453
- US-A1- 2008 125 718
- US-A1- 2014 324 024
- US-A1- 2014 343 501

## Beschreibung

Die Erfindung betrifft eine Fixiervorrichtung zur Fixierung einer Katheteranordnung mit den oberbegrifflichen Merkmalen des Anspruchs 1.

Eine derartige Fixiervorrichtung ist aus der US 9 056 186 B2 bekannt und weist eine Aufnahmeeinheit in Form eines Grundkörpers auf. Der Grundkörper ist zur Verbindung mit einer Hautfläche des Patienten vorgesehen und weist eine Aufnahmeaussparung auf. Die Aufnahmeaussparung ist zur Aufnahme eines V-förmigen Verzweigungsabschnitts der Katheteranordnung vorgesehen. Bei der bekannten Fixiervorrichtung ist die Aufnahmeaussparung derart gestaltet, dass der Verzweigungsabschnitt in Axialrichtung formschlüssig an der Aufnahmeaussparung festgelegt ist. Zudem ist eine relativ zu der Aufnahmeaussparung verlagerbare Verschlusseinheit in Form eines Deckels vorgesehen. Der Deckel ist zwischen einem Freigabezustand und einem Schließzustand verlagerbar. Im Schließzustand ist der Deckel mit dem Grundkörper verrastet und verschließt die Aufnahmeaussparung oberseitig, so dass der Verzweigungsabschnitt auch senkrecht zur Axialrichtung in der Aufnahmeaussparung festgelegt ist. Im Freigabezustand ist die Aufnahmeaussparung abschnittsweise freigegeben, so dass der Verzweigungsabschnitt in die Aufnahmeaussparung eingelegt oder aus derselben entnommen werden kann. Bei der bekannten Fixiervorrichtung ist der Deckel als separat von dem Grundkörper gefertigtes Bauteil ausgebildet und im Freigabezustand vom Grundkörper getrennt.

Aus der US 2014/324024 A1 ist eine Haltevorrichtung für Katheter bekannt, die ein erstes Rahmenelement und ein zweites Rahmenelement aufweist, das relativ zu dem ersten Rahmenelement gelenkig ist, um eine zwischen den beiden Rahmenelementen ausgebildete Halteöffnung zu verschließen und freizugeben. Zudem weist die Haltevorrichtung ein Basiselement auf, das mit dem zweiten Rahmenelement verbunden ist.

Aus der US 2014/343501 A1 ist ein Verankerungssystem für einen länglichen medizinischen Artikel bekannt, das ein Verankerungspolster und eine auf dem Verankerungspolster montierte Halterung umfasst. Die Halterung besteht aus einer Basis, einer Abdeckung und einem komprimierbaren Element mit einer Aufnahme, in die der zu haltende medizinische Artikel gelegt wird. Das Verankerungspolster ist zur Verbindung mit der Haut des Patienten eingerichtet.

Aus der US 2005/038453 A1 ist eine Fixiervorrichtung mit einer Aufnahmeeinheit, einer Aufnahmeaussparung und einer Verschlusseinheit bekannt. Die Verschlusseinheit kann zum Verschließen und Freigeben der Aufnahmeaussparung verlagert werden. Um die Verlagerung der Verschlusseinheit zu ermöglichen, ist die Aufnahmeeinheit in zwei Hälften untergliedert, die mittels eines Filmgelenks gelenkig miteinander verbunden sind. Die gesamte Fixiervorrichtung ist aus Polypropylen gefertigt.

Aus der US 2008/125718 A1 ist eine weitere Fixiervorrichtung bekannt.

Aufgabe der Erfindung ist es, eine Fixiervorrichtung der eingangs genannten Art zu schaffen, die eine einfache und sichere Handhabung und gleichzeitig einen besonders einfachen Aufbau ermöglicht.

Diese Aufgabe wird durch eine Fixiervorrichtung mit den Merkmalen des Anspruchs 1 gelöst. Erfindungsgemäß ist vorgesehen, dass die Verschlusseinheit unlösbar mit der Aufnahmeeinheit zusammengefügt ist, wobei die Aufnahmeeinheit wenigstens abschnittsweise derart formelastisch ausgebildet ist, dass die Verschlusseinheit mittels einer elastischen Deformation der Aufnahmeeinheit zwischen dem Freigabezustand und dem Schließzustand verlagerbar ist. Durch die erfindungsgemäße Lösung wird zum einen einem Verlust der Verschlusseinheit im Freigabezustand entgegengewirkt. Dies, da die Verschlusseinheit unlösbar mit der Aufnahmeeinheit zusammengefügt ist. Dadurch wird sowohl eine sichere Handhabung als auch ein besonders einfacher Aufbau der Fixiervorrichtung ermöglicht. Zum anderen ermöglicht die erfindungsgemäße Lösung gleichzeitig eine besonders einfache Verlagerung der Verschlusseinheit zwischen dem Freigabe- und dem Schließzustand. Auch dies trägt zu einer besonders einfachen und sicheren Handhabung der Fixiervorrichtung bei. Hierzu ist die Aufnahmeeinheit wenigstens abschnittsweise derart formelastisch ausgebildet, dass die Verschlusseinheit mittels einer elastischen Deformation der Aufnahmeeinheit verlagerbar ist. Dies trägt ebenfalls zu einem besonders einfachen Aufbau der Fixiervorrichtung bei. Im Sinne der Erfindung meint unlösbar zusammengefügt, dass ein zerstörungsfreies Trennen der Verschlusseinheit und der Aufnahmeeinheit nicht möglich ist. Die Aufnahmeeinheit und die Verschlusseinheit können insbesondere kraft-, form- und/oder stoffschlüssig unlösbar zusammengefügt sein. Vorzugsweise besteht eine stoffschlüssige Verbindung. Zudem ist es besonders vorteilhaft, wenn die Verschlusseinheit und die Aufnahmeeinheit einstückig zusammenhängend ausgebildet sind. Die Aufnahmeeinheit kann mittels einer entsprechenden Werkstoffwahl und/oder konstruktiven Gestaltung formelastisch ausgebildet sein. Beispielsweise kann die Aufnahmeeinheit wenigstens abschnittsweise dünnwandig ausgebildet sein und/oder aus einem formelastischen Werkstoff gefertigt sein. Die Aufnahmeeinheit kann insbesondere elastisch dehn-, scher- und/oder biegbar ausgebildet sein. Vorzugsweise ist die Aufnahmeeinheit formelastisch biegbar ausgebildet, so dass die Verschlusseinheit mittels einer elastischen Biegedeformation der Aufnahmeeinheit zwischen dem Freigabezustand und dem Schließzustand verschwenkbar ist. Vorzugsweise ist die gesamte Aufnahmeeinheit formelastisch ausgebildet. Es ist vorteilhaft, wenn die Aufnahmeeinheit eine der Aufnahmeaussparung abgewandte Rückseite aufweist, die zur Auflage auf der Hautfläche vorgesehen ist. Zur Verbindung der Aufnahmeeinheit mit der Hautfläche kann diese auf grundsätzlich bekannte Weise auf die Hautfläche aufgeklebt oder mit dieser vernäht werden. Für letztgenannte Verbindungsoption kann die Aufnahmeeinheit insbesondere flügelartig abragende Befestigungsabschnitte aufweisen, die mit jeweils einer Durchgangsbohrung als Durchlass für ein Nahtmaterial versehen sind. Die Aufnahmeaussparung kann insbesondere derart gestaltet sein, dass ein Verzweigungs- oder ein Schlauchabschnitt der Katheteranordnung im Schließzustand kraft- und/oder formschlüssig an und/oder in der Aufnahmeaussparung festgelegt ist. Vorzugsweise ist die Aufnahmeaussparung kanalförmig ausgebildet und zur Aufnahme eines Schlauchabschnitts der Katheteranordnung vorgesehen. Dabei kann die kanalförmige Aufnahmeaussparung den Schlauchabschnitt in Umfangsrichtung derart umgreifen, dass derselbe in Axialrichtung reibschlüssig an der Aufnahmeaussparung festgelegt ist. Die Verschlusseinheit kann die Aufnahmeaussparung im Schließzustand wenigstens abschnittsweise form- und/oder kraftschlüssig verschließen. Hierzu kann die Verschlusseinheit insbesondere in Form einer Rast-, Steck- oder Klemmeinheit ausgebildet sein.

Die erfindungsgemäße Lösung eignet sich in besonders vorteilhafter Weise zur patientenseitigen Fixierung eines zentralvenösen Katheters. Dessen ungeachtet kann die erfindungsgemäße Lösung auch zur Fixierung sonstiger Katheteranordnungen an einem Patienten verwendet werden.

Weiter gemäß der Erfindung ist die Aufnahmeeinheit aus einem weichelastischen Kunststoff gefertigt und die Verschlusseinheit ist aus einem im Wesentlichen formstabilen Kunststoff gefertigt. Durch die weichelastische Gestaltung der gesamten Aufnahmeeinheit können insbesondere fertigungsbedingte maßliche Abweichungen des zu fixierenden Abschnitts der Katheteranordnung ausgeglichen werden. Dies, da die Aufnahmeaussparung aufgrund der Fertigung der Aufnahmeeinheit aus weichelastischem Kunststoff ebenfalls elastisch nachgiebig ausgebildet ist. Durch die weichelastische Gestaltung der Aufnahmeeinheit wird zudem einer Irritation der an der Aufnahmeeinheit anliegenden Hautfläche entgegengewirkt. Als weichelastischer Kunststoff kann insbesondere ein Elastomer oder ein Silikonelastomer verwendet werden. Durch die Fertigung der Verschlusseinheit aus einem im Wesentlichen formstabilen Kunststoff wird insbesondere ein sicheres Verschließen der Aufnahmeaussparung im Schließzustand gewährleistet.

In weiterer Ausgestaltung der Erfindung sind die Aufnahmeeinheit und die Verschlusseinheit einstückig zusammenhängend in Form eines Mehr-Komponenten-Spritzgussbauteils aus wenigstens zwei unterschiedlichen Kunststoffen gefertigt. Verfahren zur Fertigung von Mehr-Komponenten-Spritzgussbauteilen aus Kunststoff sind grundsätzlich bekannt. Vorzugsweise ist die Fixiervorrichtung in Form eines Zwei-Komponenten-Spritzgussbauteils gefertigt. Dies ist eine besonders vorteilhafte Ausgestaltung der Erfindung. Denn zum einen ermöglicht die Zwei-Komponenten-Fertigung eine besonders zuverlässige und kostengünstig herstellbare unlösbare Fügeverbindung zwischen der Verschlusseinheit und der Aufnahmeeinheit. Zum anderen gestaltet sich die gesamte Fertigung der Fixiervorrichtung kostengünstig und einfach.

In weiterer Ausgestaltung der Erfindung ist die Aufnahmeaussparung in Form eines längserstreckten Kanals ausgebildet, der einen Längsschlitz zum Einbringen eines Schlauchabschnitts der Katheteranordnung in den Kanal aufweist, wobei eine Schlitzbreite des Längsschlitzes mittels der elastischen Deformation der Aufnahmeeinheit veränderlich ist. Der Kanal dient der Aufnahme des Schlauchabschnitts. Zum Einbringen des Schlauchabschnitts in den Kanal und zur Entnahme des Schlauchabschnitts aus dem Kanal ist der Längsschlitz vorgesehen, der sich vorzugsweise über die gesamte Länge des Kanals erstreckt. Vorzugsweise weist der Kanal wenigstens im Schließzustand eine im Wesentlichen kreiszylindrische Grundform auf, die auf eine Formgebung des zu fixierenden Schlauchabschnitts abgestimmt ist. Bei dieser Ausgestaltung ist es besonders vorteilhaft, wenn die Aufnahmeeinheit wenigstens im Bereich der Aufnahmeaussparung formelastisch ausgebildet ist. Dadurch können toleranzbedingte Formabweichungen des Schlauchabschnitts kompensiert werden. Der Kanal kann zur nochmals verbesserten Fixierung mit einer, insbesondere zacken- oder wellenförmig ausgebildeten, Profilierung versehen sein.

In weiterer Ausgestaltung der Erfindung bewirkt die Verschlusseinheit im Schließzustand eine wenigstens abschnittsweise elastische Vorspannung des Kanals in Radialrichtung des Kanals zur reibschlüssigen Fixierung des Schlauchabschnitts in Axialrichtung des Kanals. Vereinfacht ausgedrückt ist der Kanal im Schließzustand mittels der Verschlusseinheit radial zusammengedrückt. Dies ermöglicht eine besonders sichere Fixierung des Schlauchabschnitts.

In weiterer Ausgestaltung der Erfindung weist die Verschlusseinheit einen Rastabschnitt und einen komplementären Gegenrastabschnitt auf, wobei der Rastabschnitt und der Gegenrastabschnitt mittels der elastischen Deformation der Aufnahmeeinheit relativ zueinander beweglich sind. Der Rastabschnitt und der Gegenrastabschnitt sind im Schließzustand formschlüssig aneinander gehalten und im Freigabezustand voneinander freigegeben. Der Rastabschnitt kann insbesondere in Form einer Rastnase, eines Rasthakens oder dergleichen ausgebildet sein. Der Gegenrastabschnitt kann insbesondere in Form eines Rastvorsprungs, einer Rastvertiefung oder dergleichen ausgebildet sein. Vorzugsweise ist der Rastabschnitt - in Bezug auf eine Längsrichtung der Aufnahmeaussparung - einerseits der Aufnahmeaussparung festgelegt und der Gegenrastabschnitt ist andererseits der Aufnahmeaussparung festgelegt. Zur Verlagerung zwischen dem Freigabe- und dem Schließzustand können der Rastabschnitt und der Gegenrastabschnitt relativ zueinander rotatorisch und/oder translatorisch beweglich sein.

In weiterer Ausgestaltung der Erfindung sind der Rastabschnitt und der Gegenrastabschnitt um eine Schwenkachse relativ zueinander verschwenkbar, wobei die Aufnahmeeinheit einen elastischen Festkörpergelenkabschnitt aufweist, der die relative Schwenkbeweglichkeit des Rastabschnitts und des Gegenrastabschnitts ermöglicht. Die Schwenkachse ist vorzugsweise parallel zu einer Längsrichtung der Aufnahmeaussparung orientiert. Vorzugsweise ist der Festkörpergelenkabschnitt - in Bezug auf eine Hochrichtung der Fixiervorrichtung - unterhalb der Aufnahmeaussparung angeordnet. Der Festkörpergelenkabschnitt ermöglicht eine elastische Biegedeformation der Aufnahmeeinheit. Die Aufnahmeeinheit ist wenigstens im Bereich des Festkörpergelenkabschnitts elastisch ausgebildet. Dessen ungeachtet kann die gesamte Aufnahmeeinheit formelastisch ausgebildet sein. Der Festkörpergelenkabschnitt ist vorzugsweise mittels einer geometrischen Schwächung, insbesondere einer Wandstärkenreduktion, der Aufnahmeeinheit gebildet. Vorzugsweise ist die geometrische Schwächung, insbesondere die Wandstärkenreduktion, mittels der Aufnahmeaussparung bewirkt. Bei dieser Ausgestaltung der Erfindung ist es besonders vorteilhaft, wenn die Aufnahmeaussparung in Form eines längserstreckten Kanals ausgebildet ist.

Weitere Vorteile und Merkmale der Erfindung ergeben sich aus den Ansprüchen sowie aus der nachfolgenden Beschreibung eines bevorzugten Ausführungsbeispiels der Erfindung, das anhand der Zeichnungen dargestellt ist.
- Fig. 1: zeigt in schematischer Perspektivdarstellung eine Ausführungsform einer erfindungsgemäßen Fixiervorrichtung in einem Schließzustand,
- Fig. 2: in einer Ansicht entsprechend Fig. 1 die Fixiervorrichtung nach Fig. 1 in einem Freigabezustand,
- Fig. 3: in einer perspektivischen Querschnittsdarstellung die Fixiervorrichtung nach den Fig. 1 und 2 im Freigabezustand und
- Fig. 4, 5: jeweils in schematischer Seitenansicht in axialer Blickrichtung auf eine Aufnahmeaussparung die Fixiervorrichtung nach den Fig. 1 bis 3 im Schließzustand (Fig. 4) bzw. im Freigabezustand (Fig. 5).

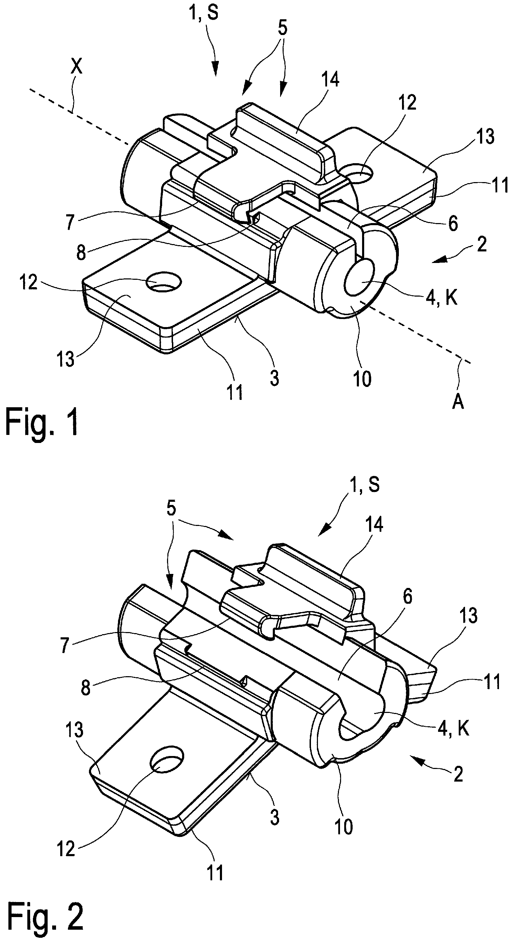

Gemäß Fig. 1 ist eine Fixiervorrichtung 1 zur Fixierung einer zeichnerisch nicht dargestellten Katheteranordnung an einem Patienten vorgesehen. Die zu fixierende Katheteranordnung ist vorliegend eine zentralvenöse Katheteranordnung. Solche Katheteranordnungen sind grundsätzlich bekannt und können ein- oder mehrlumig ausgestaltet sein und weisen wenigstens einen - im applizierten Zustand der Katheteranordnung - außerhalb des Patienten angeordneten Schlauchabschnitt auf. Dessen ungeachtet kann die Fixiervorrichtung 1 auch zur Fixierung sonstiger Katheteranordnungen verwendet werden.

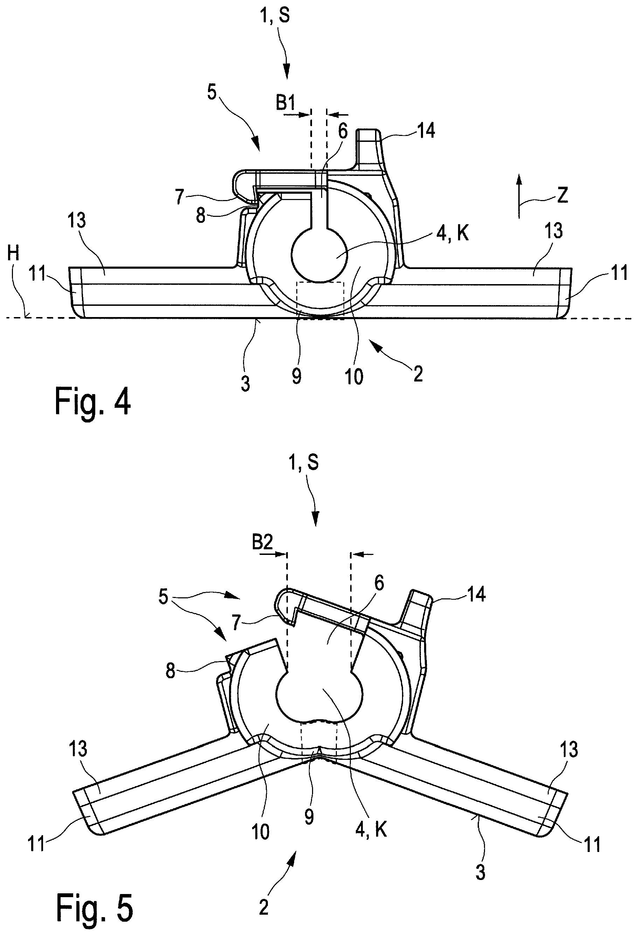

Die Fixiervorrichtung 1 weist eine Aufnahmeeinheit 2 auf, die zur Verbindung mit einer Hautfläche H (Fig. 4) des Patienten vorgesehen ist. Anhand Fig. 4 ist die Hautfläche H schematisch stark vereinfacht dargestellt. Dabei ist ersichtlich, dass die Aufnahmeeinheit 2 in der anhand Fig. 4 ersichtlichen Konfiguration mit einer Rückseite 3 flächig auf der Hautfläche H aufliegt, wobei die Aufnahmeeinheit 2 auf noch näher beschriebene Weise mit der Hautfläche H verbunden ist. Die Aufnahmeeinheit 2 weist eine Aufnahmeaussparung 4 auf. Die Aufnahmeaussparung 4 ist zur Aufnahme eines Abschnitts der Katheteranordnung vorgesehen. Vorliegend ist die Aufnahmeaussparung 1 zur Aufnahme des besagten Schlauchabschnitts der Katheteranordnung vorgesehen. Zudem weist die Fixiervorrichtung 1 eine Verschlusseinheit 5 auf, die einem Verschließen bzw. einem Freigeben der Aufnahmeaussparung 4 wenigstens in Hochrichtung Z der Fixiervorrichtung 1 dient. Zu diesem Zweck ist die Verschlusseinheit zwischen einem Freigabezustand (Fig. 2, 3, 5), in dem die Aufnahmeaussparung 4 zur Auf- und/oder Entnahme des Schlauchabschnitts der Katheteranordnung freigegeben ist, und einem Schließzustand (Fig. 1, 4), in dem die Aufnahmeaussparung 4 mittels der Verschlusseinheit 5 wenigstens abschnittsweise verschlossen ist, verlagerbar.

Vorliegend ist die Verschlusseinheit 5 unlösbar mit der Aufnahmeeinheit 2 zusammengefügt, wobei die Aufnahmeeinheit 2 wenigstens abschnittsweise derart formelastisch ausgebildet ist, dass die Verschlusseinheit 5 mittels einer elastischen Deformation der Aufnahmeeinheit 2 zwischen dem Freigabezustand und dem Schließzustand verlagerbar ist.

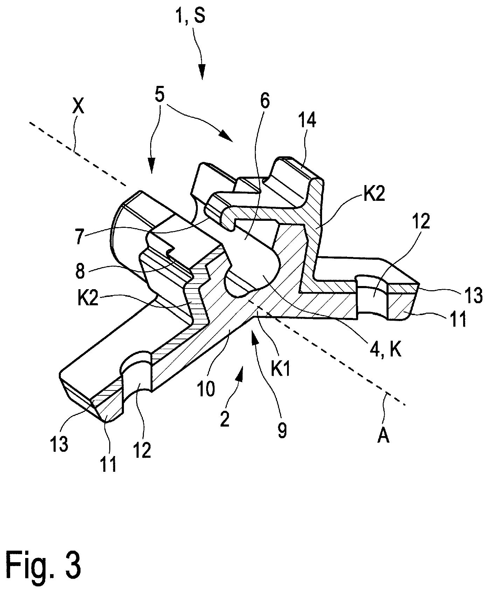

Um die wenigstens abschnittsweise formelastische Deformierbarkeit der Aufnahmeeinheit 2 zu erreichen, ist letztere vorliegend aus einem weichelastischen Kunststoff K1 gefertigt (Fig. 3). Demgegenüber ist die Verschlusseinheit 5 aus einem im Wesentlichen formstabilen Kunststoff K2 gefertigt. Als weichelastischer Kunststoff K1 ist vorliegend ein Silikonelastomer gewählt. Wie weiter insbesondere anhand der Fig. 3 erkennbar ist, sind die Aufnahmeeinheit 3 und die Verschlusseinheit 5 einstückig zusammenhängend gefertigt. Insoweit ist die gesamte Fixiervorrichtung 1 als einstückiges Bauteil in Form eines Mehr-Komponenten-Spritzgussbauteils S ausgebildet. Aufgrund der vorliegenden Gestaltung ist das Mehr-Komponenten-Spritzgussbauteil S ein Zwei-Komponenten-Spritzgussbauteil. Verfahren zur Herstellung von Mehr- oder insbesondere Zwei-Komponenten-Spritzgussbauteilen aus Kunststoff sind im Bereich der Kunststofftechnik grundsätzlich bekannt.

Die Aufnahmeaussparung 4 ist vorliegend in Form eines längserstreckten Kanals K ausgebildet. Der Kanal K weist einen Längsschlitz 6 auf, der zum Einbringen des Schlauchabschnitts in den Kanal K und/oder zur Entnahme des Schlauchabschnitts aus dem Kanal K vorgesehen ist. Dabei erstreckt sich der Längsschlitz 6 in Axialrichtung X des Kanals K über dessen gesamte Länge. Eine Schlitzbreite B1, B2 des Längsschlitzes 6 ist mittels der elastischen Deformation der Aufnahmeeinheit 2 veränderlich. Im Schließzustand weist der Längsschlitz 6 eine Schlitzbreite B1 und im Freigabezustand eine hierzu vergleichsweise größere Schlitzbreite B2 auf.

Die Verschlusseinheit 5 weist einen Rastabschnitt 7 und einen Gegenrastabschnitt 8 auf. Der Rastabschnitt ist in Form eines Rasthakens 7 ausgebildet. Der Gegenrastabschnitt ist vorliegend in Form eines Rastvorsprungs 8 ausgebildet. Im Schließzustand sind der Rastabschnitt 7 und der Gegenrastabschnitt 8 formschlüssig aneinander festgelegt. Im Freigabezustand sind der Rastabschnitt 7 und der Gegenrastabschnitt 8 demgegenüber voneinander freigegeben. Vorliegend sind der Rastabschnitt 7 und der Gegenrastabschnitt 8 um eine Schwenkachse A relativ zueinander verschwenkbar, wobei die Aufnahmeeinheit 2 einen elastischen Festkörpergelenkabschnitt 9 aufweist, der die relative Schwenkbeweglichkeit des Rastabschnitts 7 und des Gegenrastabschnitts um die Schwenkachse A ermöglicht (Fig. 3). Die Schwenkachse A und die Längsrichtung X der Aufnahmeaussparung 4 bzw. des Kanals K sind vorliegend parallel zueinander ausgerichtet. Der Festkörpergelenkabschnitt 9 ist in Hochrichtung Z unterhalb des Kanals K angeordnet und ermöglicht eine elastische Biegebeweglichkeit der Aufnahmeeinheit 2 und damit eine entsprechende Verschwenkbarkeit der Verschlusseinheit 5 zur Überführung zwischen dem Schließ- und dem Freigabezustand. Nachfolgend wird näher auf weitere funktionelle sowie konstruktive Details der Fixiervorrichtung 1 eingegangen.

Die Aufnahmeeinheit 2 weist einen Grundabschnitt 10 und zwei zu gegenüberliegenden Querseiten des Grundabschnitts 10 seitlich abragende Flügelabschnitte 11 auf. Der Grundabschnitt 10 weist eine im Wesentlichen kreiszylindrische Grundform auf, wobei der Kanal K in etwa mittig entlang der Längsrichtung X durch den Grundabschnitt 10 erstreckt ist. Jedenfalls im Schließzustand (Fig. 1, 4) weist der Kanal K eine kreisrunde Querschnittsform auf. Die beiden Flügelabschnitte 11 weisen jeweils eine plattenförmige, rechteckige Grundform auf und sind in Bezug auf die Hochrichtung Z unterhalb einer horizontal ausgerichteten Mittellängsebene des Grundabschnitts 10 angeordnet. Die Flügelabschnitte 11 weisen jeweils eine Durchgangsbohrung 12 auf. Die Durchgangsbohrungen 12 sind - in Bezug auf die Längsrichtung X - in etwa mittig an den Flügelabschnitten 11 angeordnet. Entsprechendes gilt in Bezug auf eine quer zur Längsrichtung X orientierte Anordnung der Durchgangsbohrungen 12. Die Durchgangsbohrungen 12 ermöglichen ein Befestigen der Fixiervorrichtung 1 mittels medizinischen Nahtmaterials an der Hautfläche H. Eine solche Art der Befestigung ist grundsätzlich bekannt. Bei einer nicht näher dargestellten Ausführungsform kann die Rückseite 3 zum Aufkleben auf die Hautfläche H vorbereitet sein. Der Grundabschnitt 10 ragt stirnendseitig in Längsrichtung X beidseits über die Flügelabschnitte 11 hinaus. Im Schließzustand sind die Flügelabschnitte 11 eben orientiert. Demgegenüber sind die Flügelabschnitte 11 im Freigabezustand aufgrund der elastischen Biegedeformation im Bereich des Festkörpergelenkabschnitts 9 relativ zueinander um die Schwenkachse A nach unten verschwenkt. Dabei ist der Kanal K ausgehend von der vorbeschriebenen kreisrunden Querschnittsform in Radialrichtung aufgeweitet und oberseitig aufgeklappt, so dass der Längsschlitz 6 ausgehend von der Schlitzbreite B1 auf die Schlitzbreite B2 verbreitert ist.

Die Verschlusseinheit 5 ist - vereinfacht ausgedrückt - oberseitig auf die Aufnahmeeinheit 2 aufgespritzt und einstückig zusammenhängend mit dieser verbunden. Dabei weist die Verschlusseinheit 5 zwei die Flügelabschnitte 11 flächig bedeckende Seitenabschnitte 13 auf. Die Durchgangsbohrungen 12 erstrecken sich durch die Seitenabschnitte 13. Ausgehend von den Flügelabschnitten 11 übergreifen die Seitenabschnitte 13 den Grundabschnitt 10 mittig beidseits des Kanals K, wobei der Rastabschnitt 7 an dem - in Bezug auf die Zeichenebenen der Fig. 4 und 5 - rechten Seitenabschnitt 13 und der Gegenrastabschnitt 8 an dem linken Seitenabschnitt 13 angeordnet sind. Zudem weist die Verschlusseinheit 5 einen Handhabungsabschnitt 14 auf. Dieser ist an dem rechten Seitenabschnitt 13 angeordnet und in Form eines in Hochrichtung Z abragenden Vorsprungs ausgebildet. Im Schließzustand übergreift der Rastabschnitt 7 den Kanal K oberseitig, so dass einem ungewollten Herausrutschen des Schlauchabschnitts aus dem Kanal K durch den Längsschlitz 6 entgegengewirkt ist. Zudem bewirkt die vorliegende Gestaltung der Verschlusseinheit 5, dass der Kanal K im Schließzustand in radialer Richtung elastisch vorgespannt ist, so dass der Schlauchabschnitt in Axialrichtung - d.h. in Längsrichtung X des Kanals K - reibschlüssig an dem Kanal K festgelegt ist. Dies wirkt einem ungewollten Verrutschen des Schlauchabschnitts entlang des Kanals K entgegen.

Zur Fixierung der Katheteranordung wird der Schlauchabschnitt im Freigabezustand der Verschlusseinheit 5 durch den Längsschlitz 6 in den Kanal K eingebracht. Erforderlichenfalls kann die Aufnahmeeinheit 2 hierzu über den anhand der Fig. 2, 3 sowie 5 ersichtlichen Deformationszustand hinaus elastisch deformiert werden, so dass der Schlauchabschnitt in einfacher Weise an dem Rastabschnitt 7 vorbei durch den Längsschlitz 6 in den Kanal K bewegt werden kann. Zum Überführen der Verschlusseinheit 5 in den Schließzustand (Fig. 1, 4) wird die Fixiervorrichtung 1 mittels manuellem Druck im Bereich des Handhabungsabschnitts 14 gegen die Hautfläche H nach unten gedrückt. Dies bewirkt eine biegeelastische Deformation der Aufnahmeeinheit 2 im Bereich des Festkörpergelenkabschnitts 9, wodurch der Rastabschnitt 7 relativ zu dem Gegenrastabschnitt 8 um die Schwenkachse A verschwenkt wird, bis eine formschlüssige Rastverbindung hergestellt ist. Dabei kann die Fixiervorrichtung 1 auf die vorbeschriebene Weise an der Hautfläche H vernäht sein, was jedoch nicht zwingend der Fall sein muss. Zur Entnahme des Schlauchabschnitts aus dem Kanal K kann die Verschlusseinheit 5 mittels einer Betätigung des Handhabungsabschnitts 14 und/oder mittels manuellen Herunterdrückens der Seitenabschnitte 13 in den Freigabezustand überführt werden. Dabei erlaubt die erfindungsgemäße Gestaltung der Fixiervorrichtung 1 grundsätzlich eine einhändige Bedienung, was die Handhabung wesentlich erleichtert.

## Patentansprüche

1. Fixiervorrichtung (1) zur Fixierung einer Katheteranordnung an einem Patienten, aufweisend
- eine Aufnahmeeinheit (2), die zur Verbindung mit einer Hautfläche (H) des Patienten vorgesehen ist,
- eine Aufnahmeaussparung (4), die an der Aufnahmeeinheit (2) angeordnet ist und zur Aufnahme eines Abschnitts der Katheteranordnung vorgesehen ist,
- und eine Verschlusseinheit (5), die relativ zu der Aufnahmeaussparung (4) verlagerbar ist zwischen einem Freigabezustand, in dem die Aufnahmeaussparung (4) zur Auf- und/oder Entnahme des Abschnitts der Katheteranordnung freigegeben ist, und einem Schließzustand, in dem die Aufnahmeaussparung (4) mittels der Verschlusseinheit (5) wenigstens abschnittsweise verschlossen ist,
- **dadurch gekennzeichnet, dass** die Verschlusseinheit (5) unlösbar mit der Aufnahmeeinheit (2) zusammengefügt ist, wobei die Aufnahmeeinheit (2) wenigstens abschnittsweise derart formelastisch ausgebildet ist, dass die Verschlusseinheit (5) mittels einer elastischen Deformation der Aufnahmeeinheit (2) zwischen dem Freigabezustand und dem Schließzustand verlagerbar ist, wobei die Aufnahmeeinheit (2) aus einem weichelastischen Kunststoff (K1) gefertigt ist, und wobei die Verschlusseinheit (5) aus einem im Wesentlichen formstabilen Kunststoff (K2) gefertigt ist.

2. Fixiervorrichtung (1) nach Anspruch 1, **dadurch gekennzeichnet, dass** die Aufnahmeeinheit (2) und die Verschlusseinheit (5) einstückig zusammenhängend in Form eines Mehr-Komponenten-Spritzgussbauteils (S) aus wenigstens zwei unterschiedlichen Kunststoffen (K1, K2) gefertigt sind.

3. Fixiervorrichtung (1) nach einem der vorhergehenden Ansprüche, **dadurch gekennzeichnet, dass** die Aufnahmeaussparung (4) in Form eines längserstreckten Kanals (K) ausgebildet ist, der einen Längsschlitz (6) zum Einbringen eines Schlauchabschnitts der Katheteranordnung in den Kanal (K) aufweist, wobei eine Schlitzbreite (B1, B2) des Längsschlitzes (6) mittels der elastischen Deformation der Aufnahmeeinheit (2) veränderlich ist.

4. Fixiervorrichtung (1) nach Anspruch 3, **dadurch gekennzeichnet, dass** die Verschlusseinheit (5) im Schließzustand eine wenigstens abschnittsweise elastische Vorspannung des Kanals (K) in Radialrichtung des Kanals (K) zur reibschlüssigen Fixierung des Schlauchabschnitts in Axialrichtung (X) des Kanals (K) bewirkt.

5. Fixiervorrichtung (1) nach einem der vorhergehenden Ansprüche, **dadurch gekennzeichnet, dass** die Verschlusseinheit (5) einen Rastabschnitt (7) und einen komplementären Gegenrastabschnitt (8) aufweist, wobei der Rastabschnitt (7) und der Gegenrastabschnitt (8) mittels der elastischen Deformation der Aufnahmeeinheit (2) relativ zueinander beweglich sind.

6. Fixiervorrichtung (1) nach Anspruch 5, **dadurch gekennzeichnet, dass** der Rastabschnitt (7) und der Gegenrastabschnitt (8) um eine Schwenkachse (A) relativ zu einander verschwenkbar sind, wobei die Aufnahmeeinheit (2) einen elastischen Festkörpergelenkabschnitt (9) aufweist, der die relative Schwenkbeweglichkeit des Rastabschnitts (7) und des Gegenrastabschnitts (8) ermöglicht.

## Claims

1. Fixing device (1) for fixing a catheter assembly to a patient, having
- a receiving unit (2), which is provided for connection to a skin surface (H) of the patient,
- a receiving recess (4), which is arranged on the receiving unit (2) and is provided to receive a portion of the catheter assembly,
- and a closure unit (5), which is movable relative to the receiving recess (4) between an opened state, in which the receiving recess (4) is open for receipt and/or removal of the portion of the catheter assembly, and a closed state, in which the receiving recess (4) is at least partially closed by means of the closure unit (5),
- **characterized in that** the closure unit (5) is joined non-releasably to the receiving unit (2), wherein the receiving unit (2) is designed at least in part with shape elasticity, such that the closure unit (5) is movable between the opened state and the closed state by means of an elastic deformation of the receiving unit (2), wherein the receiving unit (2) is made of a flexible plastic (K1), and **in that** the closure unit (5) is made of a substantially dimensionally stable plastic (K2).

2. Fixing device (1) as claimed in claim 1, **characterized in that** the receiving unit (2) and the closure unit (5) are made as one continuous piece in the form of a multi-component injection molding (S) from at least two different plastics (K1, K2).

3. Fixing device (1) as claimed in one of the preceding claims, **characterized in that** the receiving recess (4) is designed in the form of an elongate channel (K), which has a longitudinal slit (6) for the introduction of a hose portion of the catheter assembly into the channel (K), wherein a slit width (B1, B2) of the longitudinal slit (6) is modifiable by means of the elastic deformation of the receiving unit (2).

4. Fixing device (1) as claimed in claim 3, **characterized in that** the closure unit (5), in the closed state, causes an at least partial elastic prestressing of the channel (K) in the radial direction of the channel (K), in order to frictionally fix the hose portion in the axial direction (X) of the channel (K).

5. Fixing device (1) as claimed in one of the preceding claims, **characterized in that** the closure unit (5) has a latching portion (7) and a complementary mating latching portion (8), wherein the latching portion (7) and the mating latching portion (8) are movable relative to each other by means of the elastic deformation of the receiving unit (2).

6. Fixing device (1) as claimed in claim 5, **characterized in that** the latching portion (7) and the mating latching portion (8) are pivotable relative to each other about a pivot axis (A), wherein the receiving unit (2) has an elastic flexure portion (9), which permits the relative pivotability of the latching portion (7) and of the mating latching portion (8).

## Revendications

1. Dispositif de fixation (1) pour fixer un agencement de cathéter sur un patient, présentant
- une unité de réception (2) prévue pour être reliée à une surface cutanée (H) du patient,
- un évidement de réception (4) agencé sur l'unité de réception (2) et prévu pour recevoir une section de l'agencement de cathéter,
- et une unité de fermeture (5), qui peut être déplacée par rapport à l'évidement de réception (4) entre un état de libération, dans lequel l'évidement de réception (4) est libéré pour la mise en place et/ou le retrait de la section de l'agencement de cathéter, et un état de fermeture, dans lequel l'évidement de réception (4) est fermé au moins par sections au moyen de l'unité de fermeture (5),
- **caractérisé en ce que** l'unité de fermeture (5) est assemblée de manière indémontable à l'unité de réception (2), l'unité de réception (2) étant réalisée, au moins par sections, de manière à être élastique de forme, de telle sorte que l'unité de fermeture (5) puisse être déplacée entre l'état de libération et l'état de fermeture au moyen d'une déformation élastique de l'unité de réception (2), l'unité de réception (2) étant fabriquée dans une matière plastique souple et élastique (K1) et l'unité de fermeture (5) étant fabriquée dans une matière plastique essentiellement indéformable (K2).

2. Dispositif de fixation (1) selon la revendication 1, **caractérisé en ce que** l'unité de réception (2) et l'unité de fermeture (5) sont fabriquées d'un seul tenant sous la forme d'un composant moulé par injection à plusieurs composants (S) à partir d'au moins deux matières plastiques différentes (K1, K2).

3. Dispositif de fixation (1) selon l'une quelconque des revendications précédentes, **caractérisé en ce que** l'évidement de réception (4) est réalisé sous la forme d'un canal allongé (K) qui présente une fente longitudinale (6) pour l'introduction d'une section de tuyau de l'agencement de cathéter dans le canal (K), une largeur de fente (B1, B2) de la fente longitudinale (6) pouvant être modifiée au moyen de la déformation élastique de l'unité de réception (2).

4. Dispositif de fixation (1) selon la revendication 3, **caractérisé en ce que** l'unité de fermeture (5), à l'état fermé, provoque une précontrainte au moins par sections élastique du canal (K) dans la direction radiale du canal (K) pour la fixation par friction de la section de tuyau dans la direction axiale (X) du canal (K).

5. Dispositif de fixation (1) selon l'une quelconque des revendications précédentes, **caractérisé en ce que** l'unité de fermeture (5) présente une section d'encliquetage (7) et une section d'encliquetage complémentaire (8), la section d'encliquetage (7) et la section d'encliquetage complémentaire (8) pouvant se déplacer l'une par rapport à l'autre au moyen de la déformation élastique de l'unité de réception (2).

6. Dispositif de fixation (1) selon la revendication 5, **caractérisé en ce que** la section d'encliquetage (7) et la section d'encliquetage complémentaire (8) peuvent pivoter l'une par rapport à l'autre autour d'un axe de pivotement (A), l'unité de réception (2) présentant une section d'articulation solide élastique (9) qui permet la mobilité pivotante relative de la section d'encliquetage (7) et de la section d'encliquetage complémentaire (8).
